# EUROPÄISCHE PATENTSCHRIFT

(11) **EP 1 747 264 B1**
(45) Veröffentlichungstag und Bekanntmachung des Hinweises auf die Patenterteilung: **12.09.2012**
(21) Anmeldenummer: 05743106.6
(22) Anmeldetag: 09.05.2005
(51) Int. Cl.: C12N 5/074, C12N 5/00, A61L 27/38, A61K 35/39

(54) **MULTIZELLULÄRE GEWEBE- UND ORGANKULTURSYSTEME**
MULTICELLULAR TISSUE AND ORGAN CULTURE SYSTEMS
SYSTEMES DE CULTURE TISSULAIRE ET ORGANIQUE MULTICELLULAIRES

(30) Priorität: 21.05.2004 DE 102004025081
(43) Veröffentlichungstag der Anmeldung: 31.01.2007
(73) Patentinhaber: Fraunhofer-Gesellschaft zur Förderung der angewandten Forschung e.V., 80686 München (DE)
(72) Erfinder: KRUSE, Charli, 23923 Herrnburg (DE); FUHR, Günter, 13187 Berlin (DE)
(74) Vertreter: Katzameyer, Michael
(86) Internationale Anmeldenummer: PCT/EP2005/004996
(87) Internationale Veröffentlichungsnummer: WO 2005/113747

(56) Entgegenhaltungen:
- WO-A-00/78929
- WO-A-2005/001072
- WO-A-2005/097975
- RAMIYA VIJAYAKUMAR K ET AL: "Reversal of insulin-dependent diabetes using islets generated in vitro from pancreatic stem cells" NATURE MEDICINE, NATURE AMERICA, NEW YORK, US, Bd. 6, Nr. 3, März 2000 (2000-03), Seiten 278-282, XP000864764 ISSN: 1078-8956
- TOKORO TAKAMASA ET AL: "Differentiation of acinar cells into acinoductular cells in regenerating rat pancreas." PANCREATOLOGY : OFFICIAL JOURNAL OF THE INTERNATIONAL ASSOCIATION OF PANCREATOLOGY (IAP) ... [ET AL.]. 2003, Bd. 3, Nr. 6, 2003, Seiten 487-496, XP008059087 ISSN: 1424-3903
- WEISSMAN I L ET AL: "Stem and progenitor cells: origins, phenotypes, lineage commitments, and transdifferentiations." ANNUAL REVIEW OF CELL AND DEVELOPMENTAL BIOLOGY. 2001, Bd. 17, 2001, Seiten 387-403, XP002366258 ISSN: 1081-0706
- KRUSE C ET AL: "Pluripotency of adult stem cells derived from human and rat pancreas" APPL PHYS A; APPLIED PHYSICS A: MATERIALS SCIENCE AND PROCESSING NOVEMBER 2004, Bd. 79, Nr. 7, November 2004 (2004-11), Seiten 1617-1624, XP002331149
- ANDERSON N L AND ANDERSON N G: "The human plasma proteome. History, character, and diagnostic prospects" MOLECULAR & CELLULAR PROTEOMICS, ASBBM, BIRMINGHAM, AL, US, Bd. 1, Nr. 11, 2002, Seiten 845-867, XP002986053 ISSN: 1535-9476
- LAKSHMANAN J: "Nerve growth factor levels in mouse serum: variations due to stress." NEUROCHEMICAL RESEARCH. APR 1987, Bd. 12, Nr. 4, April 1987 (1987-04), Seiten 393-397, XP008059178 ISSN: 0364-3190
- TAI M -H ET AL: "Role of pancreatic stem cells in the emergence of pancreatic stellate cells and fibroblast-like cells in chronic pancreatitis." PANCREAS, Bd. 27, Nr. 4, November 2003 (2003-11), Seiten 413-414, XP008059097 & MEETING OF THE AMERICAN PANCREATIC ASSOCIATION; CHICAGO, IL, USA; NOVEMBER 06-07, 2003 ISSN: 0885-3177
- TAKATSUGU ET AL: STEM CELLS, vol. 20, 2002, pages 41-49,
- ITSKOVITZ-ELDOR J ET AL: MOLECULAR MEDICINE, vol. 6, 2000, pages 88-95,
- CONLEY BROCK J ET AL: "Derivation, propagation and differentiation of human embryonic stem cells.", THE INTERNATIONAL JOURNAL OF BIOCHEMISTRY & CELL BIOLOGY APR 2004 LNKD- PUBMED:15010323, vol. 36, no. 4, April 2004 (2004-04), pages 555-567, ISSN: 1357-2725

## Beschreibung

Die Erfindung betrifft Verfahren zur Herstellung multizellulärer Gewebe- und Organkultursysteme.

Zur Wiederherstellung oder Übernahme der Funktion eines geschädigten oder fehlenden Gewebes oder Organs ist die Transplantation natürlicher Gewebe oder Organe von einem anderen Spender oder, falls möglich, dem betroffenen Individuum selbst eine seit langem bekannte und etablierte Praxis. Aufgrund des ständigen Mangels an geeigneten Spendergeweben und -organen und anderer Nachteile natürlicher Gewebe, z.B. Abstoßungsreaktionen und das Risiko einer Krankheitsübertragung vom Spender auf den Empfänger, richten sich viele Anstrengungen auf die Herstellung künstlicher Gewebe und Organe als Alternative.

Eine übliche Vorgehensweise ist dabei die Bereitstellung einer Träger- oder Matrixstruktur aus körperverträglichem Material, die mit differenzierten Zellen des Zielgewebes besiedelt wird, und die Kultivierung der Zellen *in vitro,* bis eine eine gewebeartige Zellstruktur entstanden ist. Die differenzierten Zellen werden entweder aus Kulturen von explantierten Gewebeproben oder aus Stammzellen, die zur Differenzierung angeregt wurden, erhalten. Die Verwendung von Stammzellen erlaubt in vielen Fällen eine schnellere Herstellung größerer Mengen der gewünschten Zellen. Herkömmlicherweise werden dabei jeweils reine Zellpopulationen eines bestimmten Typs hergestellt. Die meisten der im Stand der Technik bekannten in vitro-Organe oder in vitro-Gewebe sind insofern nachteilig, als sie selbst nach Implantation und längerer Verweilzeit im Körper nicht die Gewebestruktur aufweisen bzw. ausbilden, die dem morphologischen Aufbau des nativen Gewebes oder Organs entspricht. Dies gilt in der Regel selbst dann, wenn die Trägermatrix mit mehreren unterschiedlichen Populationen von gewebetypischen Zellen besiedelt wurde.

Ein anderer Ansatz zur Behandlung von degenerativen Erkrankungen bzw. Schädigungen von Geweben und Organen unter Verwendung von Stammzellen besteht darin, die Stammzellen und/oder davon abgeleitete differenzierte Zellen direkt in das geschädigte Gewebe/Organ zu implantieren, um dort vermehrt zu werden und zu einer Regeneration des geschädigten Gewebes/Organs zu führen. Auch hier steht herkömmlicherweise die Implantation bzw. Regeneration eines bestimmten Zelltyps im Vordergrund. Das Problem ist jedoch, daß bei sehr vielen Degenerationserscheinungen bzw. Schädigungen verschiedener Organe immer eine Vielzahl von Zellen beteiligt ist, so sind z.B. in der Haut neben Fibroblasten oft auch Keratinozyten, Epithelzellen und Blutgefäße mit betroffen. Bei Nervenschädigungen müssen oft auch Gliazellen mit ersetzt werden und Muskelschädigungen bedeuten oft auch Zerstörungen der sie begleitenden Nerven.

Demgemäß besteht eine Aufgabe der Erfindung darin, verbesserte multizelluläre Gewebe- und Organkultursysteme bereitzustellen, insbesondere humane Systeme, die aus mehreren verschiedenen Zelltypen aufgebaut sind und natürliche Gewebe- bzw. Organstrukturen aufweisen oder das Potenzial zu deren Entwicklung besitzen.

B. Conley et al., The International Journal of Biochemistry & Cell Biology, 36, 555-567 (2004), beschreiben die Herstellung multizellulärer Gewebe- und Organkultursysteme aus humanen embryonalen Stammzellen. Dieser Ansatz ist jedoch aus ethischen Gründen bedenklich und aufgrund gesetzlicher Regelungen zum Schutz von Embryos in vielen Ländern nicht praktikabel.

Die vorliegende Erfindung beruht auf dem Befund, daß multipotente oder pluripotente adulte Stammzellen, wie sie aus exokrinem Drüsengewebe gewonnen werden können (WO 2005/001072), mit einfachen Mitteln zur Aggregierung und Differenzierung in dreidimensionale Zellaggregate, sogenannte organoide Körper oder Organoid Bodies, veranlasst werden können, welche bereits ohne Zugabe spezieller Differenzierungsfaktoren ein Spektrum verschiedener Zelltypen enthalten. Die organoiden Körper wachsen bei ausreichender Nährstoffversorgung ständig weiter und entwickeln gewebe- oder organartige Strukturen, in diesem Stadium werden sie auch als Gewebekörper bezeichnet. Aufgrund ihres multizellulären Aufbaus und der bereits vorhandenen Anlage zur Ausbildung gewebe- und organartiger Strukturen sind diese organoiden Körper das ideale Ausgangsmaterial zur Herstellung von multizellulären Gewebe -und Organkultursystemen, deren Struktur der von nativen Geweben bzw. Organen weitgehend entspricht oder die im Körper zur Bildung solcher nativer Strukturen führen.

Somit werden die obengenannten Aufgaben erfindungsgemäß gelöst durch die Verfahren zur Herstellung multizellulärer Gewebe- und Organkultursysteme gemäß den Ansprüchen 1-17.

Zur Bildung der erfindungsgemäß verwendeten organoiden Körper oder Organoid Bodies werden multipotente oder pluripotente adulte Stammzellen verwendet. Vorzugsweise werden diese pluripotenten Stammzellen aus exokrinem Drüsengewebe isoliert.

Das exokrine Drüsengewebe kann von einem erwachsenen Individuum oder juvenilen Individuum stammen. Der Begriff "adult", wie in der vorliegenden Anmeldung verwendet, bezieht sich somit auf das Entwicklungsstadium des Ausgangsgewebes und nicht auf dasjenige des Donors, aus dem das Gewebe stammt. "Adulte" Stammzellen sind nicht-embryonale Stammzellen.

Vorzugsweise wird das exokrine Drüsengewebe aus einer Speicheldrüse, Tränendrüse, Talgdrüse, Schweißdrüse, aus Drüsen des Genitaltrakts, einschließlich Prostata, oder aus gastrointestinalem Gewebe, einschließlich Pankreas, oder sekretorischem Gewebe der Leber isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Die aus solchen Quellen erhaltenen adulten Stammzellen sind leicht zu isolieren und in einer stabilen Langzeitkultur ohne Feederzellschicht oder spezielle Zusätze im weitgehend undifferenzierten Zustand zu halten. Der Begriff Feederzellen, wie hier verwendet, umfasst alle Zellen, die das Wachstum der eigentlich zu kultivierenden Zellen dadurch fördern, dass sie Wachstumsfaktoren freisetzen und/oder eine extrazelluläre Matrix bereitstellen, bzw. die Differenzierung der Stammzellkultur verhindern.

Diese adulten Stammzellen können ohne Zusatz spezieller Wachstums- oder Differenzierungsfaktoren auf einfache Weise zur Differenzierung angeregt werden, indem sie unter räumlichen Bedingungen kultiviert werden, welche für einen dreidimensionalen Kontakt der Zellen sorgen. In einer bevorzugten Ausführungsform sind diese Bedingungen die Kultivierung in hängenden Tropfen, wie sie für embryonale Stammzellen bereits beschrieben wurde (Wobus et al., Biomed. Biochim. Acta 47:965-973 (1988). Dieses Verfahren wird nachfolgend in den Beispielen noch näher beschrieben. Es versteht sich jedoch, dass alternative Kultivierungsverfahren, die für den gewünschten dreidimensionalen Kontakt der Zellen sorgen und dem Fachmann bekannt und verfügbar sind, ebenso verwendet werden können. Beispiele für solche alternativen Verfahren sind die Kultivierung in bewegter Suspensionskultur, die Kultivierung in einem elektromagnetischen Feldkäfig oder Laser-Tweezer, die Aussaat nicht resuspendierter Zellen der Primärkultur, oder die Kultivierung auf Oberflächen, an denen die Zellen nicht oder schlecht haften. Solche Oberflächen können z.B. Glas, Polystyrol oder mit einer Anti-Adhäsionsschicht behandelte Oberflächen, z.B. PTFE- oder Poly-HEMA-beschichtete Oberflächen, sein.

Unter diesen Bedingungen entwickeln sich spontan dreidimensionale Zellverbände oder Zellaggregate, welche in Anlehnung an bereits für embryonale Stammzellen beschriebene "embryoid bodies" als "organoid bodies" oder organoide Körper bezeichnet wurden. Diese organoiden Körper oder Organoid Bodies können in Suspensionskulturen oder Adhäsionskulturen überführt und weiter kultiviert werden. Bei ausreichender Nährstoffversorgung wachsen diese organoiden Körper weiter und können Durchmesser von einigen Millimetern oder mehr erreichen. Diese großen Organoid Bodies zeigen eine gewebeartige Struktur und werden in diesem Stadium zur Unterscheidung von den einfachen Zellaggregaten auch als "Gewebekörper" bezeichnet. Bringt man die Organoid Bodies wieder in Oberflächenkultur, entsteht aus auswachsenden einzelnen Zellen eine zelluläre Monoschicht, aus der Multilayerbereiche hervorgehen, aus denen spontan sekundäre Organoid Bodies mit vergleichbaren Eigenschaften wie denjenigen der primären Organoid Bodies gebildet werden. Die erfindungsgemäßen Organoid Bodies können z.B. bei der Temperatur des flüssigen Stickstoffs eingefroren gelagert werden, ohne ihre Lebensfähigkeit, Vermehrungsfähigkeit, Wachstumsfähigkeit und Differenzierbarkeit zu verlieren.

Die organoiden Körper enthalten verschiedene Zelltypen aller drei Keimblätter. Differenzierte Zellen, die in den organoiden Körpern enthalten sein können, umfassen Knochenzellen (Osteoblasten und Osteoclasten), Chondrozyten, Adipozyten, Fibroblasten (z.B. Haut- und Sehnenfibroblasten), Muskelzellen, Endothelzellen, Epithelzellen, hematopoetische Zellen, sensorische Zellen, endokrine und exokrine Drüsenzellen, Gliazellen, neuronale Zellen, Oligodendrozyten, Blutzellen, Darmzellen, Herz-, Lungen-, Leber-, Nieren- oder Pankreaszellen, sind jedoch nicht darauf beschränkt.

Die verschiedenen differenzierten Zelltypen können durch histologische, immunozytochemische und elektronenmikroskopische Techniken identifiziert und charakterisiert werden.

Das Vorhandensein eines bestimmten Zelltyps kann z.B. anhand der speziellen Morphologie dieses Zelltyps oder über den Nachweis von zelltypspezifischen Markersubstanzen festgestellt werden. Einige Beispiele für zelltypspezifische Marker werden im folgenden genannt, sind jedoch keineswegs als beschränkend anzusehen: PGP 9.5 und NF für Nervenzellen, S 100 und GFAP für Gliazellen, SMA für Muskelzellen (bzw. Myofibroblasten), Kollagen Typ II für Knorpelzellen, Amylase und Trypsin für exokrine Drüsenzellen, Insulin für endokrine Drüsenzellen, Vigilin für stark translatierende Zellen und Cytokeratin für epidermale Zellen, Kollagen Typ 11 für Chondrozyten, Osteonektin für Osteoblasten und Vorläuferzellen, Osteocalcin für reife Osteoblasten, CD45, CD34, CD13 für hematopoetische Zellen, cTNI ("cardiac troponin I"), cTNT ("cardiac troponin T") und ANF ("atrial natriuretic factor") für Kardiomyozyten, Kollagenase 1 und TIMP-1 ("tissue inhibitor of metalloproteinase I") für Fibroblasten, Skelett-Alpha-Aktin und Tropomyosin für gestreifte Muskelzellen, Lipoproteinlipase (LPL) für Adipozyten, Alpha-Fetoprotein für Leberzellen. Eine sehr große Anzahl solcher Markersubstanzen ist im Stand der Technik bekannt.

Diese Markersubstanzen können z.B durch Bindung an einen spezifischen Bindungspartner, der mit einer nachweisbaren Gruppe konjugiert ist, nachgewiesen werden. Die nachweisbare Gruppe kann z.B. ein Farbstoff, Fluoreszenzfarbstoff, eine radiaktive Markierung, Enzymmarkierung, Lumineszenzmarkierung, Magnetresonanzmarkierung oder eine andere im Stand der Technik bekannte Markierung sein. Falls die Markersubstanz ein Protein ist, wird der Bindungspartner vorzugsweise ein markierter oder markierbarer Antikörper sein. Solche markierten Antikörper sind bereits für eine Vielzahl von Markersubstanzen bekannt und können entweder im Handel bezogen oder unschwer nach bekannten Verfahren hergestellt werden. Gegebenenfalls können auch markierte oder markierbare sekundäre Antikörper verwendet werden. Einige typische Proteinmarker für z.B. Nerven- und Gliazellen, Muskelzellen und sekretorische Zellen sowie immunhistochemische Nachweismethoden dafür werden im Beispielsabschnitt beschrieben.

Obwohl zur Differenzierung der Stammzellen grundsätzlich keine externen Differenzierungsfaktoren notwendig sind, können solche Differenzierungsfaktoren vorteilhaft eingesetzt werden, um gezielt größere Mengen eines bestimmten Zelltyps herzustellen bzw. um organoide Körper mit einer bestimmten Zelltypzusammensetzung zu erzeugen.

Differenzierungsfaktoren und/oder Wachstumsfaktoren sind im Stand der Technik bekannt und umfassen z.B. bFGF ("basic fibroblast growth factor") zur verstärkten Bildung von Herzzellen und Fibroblasten, VEGF ("vascular endothelial growth factor"), DMSO und Isoproterenol, Fibroblastenwachstumsfaktor 4 (FGF4), Hepatozyten-Wachstumsfaktor (HGF) zur verstärkten Bildung von Herz- und Leberzellen, TGF-betal ("transforming growth factor betal") zur verstärkten Bildung von Herzzellen, EGF ("epidermal growth factor") zur verstärkten Bildung von Haut- und Herzzellen, KGF ("keratinocyte growth factor") (machmal zusammen mit Cortison) zur Bildung von Keratinozyten, Retinsäure zur verstärkten Bildung von Nerven-, Herz- und Nierenzellen, beta-NGF ("beta nerve growth factor") zur verstärkten Bildung von Gehirn-, Leber-, Pankreas- und Nierenzellen, BMP-4 ("bone morphogenic protein 4") und Activin-A zur Bildung mesodermaler Zellen generell, sind jedoch keineswegs darauf beschränkt. In Anbetracht der reichhaltigen Literatur zu diesem Thema (siehe z.B: *"*Adult Stem Cells", Hrsg. K. Turksen, Human Press, 2004) wird der Fachmann weitere geeignete Faktoren, je nach Art der Zellen bzw. Zellkombinationen, unschwer identifizieren und gegebenenfalls einsetzen können.

Die jeweils geeigneten Differenzierungsfaktoren und/oder Wachstumsfaktoren können sich in im wesentlichen reiner Form gelöst oder immobilisiert in dem Kulturmedium befinden. Bei der Verwendung von immobilisierten Differenzierungsfaktoren befinden sich diese vorzugsweise auf einem beweglichen, relativ zu den Stammzellen oder organoiden Körpern positionierbaren Träger. Vorteilhafterweise kann damit eine gezielte Differenzierung einzelner Zellen oder Zellgruppen erreicht werden. Der Träger ist beispielsweise ein geeignetes synthetisches Substrat oder eine biologische Zelle oder Zellmembran, auf deren Oberfläche sich die Differenzierungsfaktoren befinden.

Alternativ können dem Kulturmedium auch Überstände vom Zell- oder Gewebekulturen, welche die gewünschten Differenzierungsfaktoren und/oder Wachstumsfaktoren enthalten, zugegeben werden, oder die organoiden Körper oder Stammzellen können mit geeigneten differenzierten Zellen oder Geweben, welche diese Differenzierungsfaktoren und/oder Wachstumsfaktoren produzieren, cokultiviert werden. Hierbei kann gegebenfalls auch eine Kryokonservierung dieser cokultivierten Mischung von Vorteil sein.

Eine noch weitere Möglichkeit ist die Aktivierung oder Stimulierung von mindestens einem Gen, das in die Differenzierung der Stammzellen zu den gewünschten differenzierten Zellen involviert ist, so daß diese die nötigen Differenzierungsfaktoren selbst in verstärktem Maße bilden.

In einem fortgeschrittenen Stadium der Differenzierung weisen die organoiden Körper gewebe- oder organähnliche Strukturen auf, die von zwei oder oder mehr verschiedenen Zelltypen gebildet werden (vgl. Fig. 2-6). Solche Strukturen sind z.B. neuromuskuläre Strukturen (Fig. 5) oder Glia-Nervenzell-Strukturen (Fig. 6) oder Hautzellstrukturen. Durch Kultivierung der organoiden Körper in einem Medium mit speziellen Differenzierungsfaktoren und/oder Wachstumsfaktoren kann die Ausbildung gewünschter Strukturen gefördert werden.

In einer bevorzugten Ausführungsform sind die Differenzierungsfaktoren und/oder Wachstumsfaktoren dabei auf einem beweglichen Träger immobilisiert wie oben beschrieben und es werden speziell die Zellgruppen, welche diese Strukturen aufweisen, mit den benötigten Faktoren versorgt.

In einer anderen bevorzugten Ausführungsform werden die gewebe -oder organähnlichen Strukturen aus den organoiden Körpern entnommen und dann unter geeigneten Bedingungen zur Aufrechterhaltung und/oder Entwicklung der Strukturen, einschließlich der Versorgung mit den geeigneten Differenzierungs- und Wachstumsfaktoren auf eine der oben beschriebenen Arten, weiter kultiviert bis eine gewünschte Zellmenge und/oder ein gewünschtes Entwicklungsstadium erreicht ist. Die Entnahme kann beispielsweise mit Hilfe langsamer Mikromanipulationstechniken, wie sie z.B. in der DE 103 07 487.2. beschrieben sind, erfolgen.

Grundsätzlich gibt es zur Herstellung der multizellulären Gewebe- oder Organkultursysteme mehrere Vorgehensweisen. Bei der einfachsten Verfahrensführung werden die aus der Stammzellkultur erhaltenen organoiden Körper einfach unter ausreichender Nährstoffversorgung und gegebenenfalls Versorgung mit speziellen Differenzierungs- und Wachstumsfaktoren, so lange weiterkultiviert, bis die gewünschte Menge und/oder das gewünschte Entwicklungsstadium erreicht ist. Alternativ kann es vorteilhaft sein, vollständige organoide Körper eines Entwicklungsstadiums oder Teile davon (z.B. mit gewünschten Strukturen) mit vollständigen organoiden Körpern eines anderen Entwicklungsstadiums oder Teilen davon (z.B. mit gewünschten Strukturen) und/oder davon abgeleiteten differenzierten Einzelzellen und/oder undifferenzierten adulten Stammzellen gemeinsam zu kultivieren. Damit könnte beispielsweise eine schnellere Vermehrung gewünschter Zellen und/oder eine schnellere oder spzifischere Entwicklung gewünschter Strukturen erreicht werden. In diesem Falle haben die verschiedenen organoiden Körper oder Teile davon und/oder die differenzierten oder undifferenzierten Zellen vorzugsweise die gleiche Herkunft.

Das erfindungsgemäß hergestellte Gewebe- oder Organkultursystem umfasst eine physiologisch verträgliche Matrix oder ein physiologisch verträgliches Trägersystem für die Zellen. Besonders bevorzugt ist die Matrix oder das Trägersystem im Körper abbaubar. Im Stand der Technik ist eine große Vielfalt solcher Matrices und Trägermaterialien bekannt (siehe z.B. WO 2004/7029230, WO 03/029446) und teilweise auch im Handel erhältlich.

Einige nicht-beschränkende Beispiele geeigneter Materialien sind körperverträgliche Kunststoffe wie Nylon, Polycarbonat, Polytetrafluorethylen etc. und insbesondere physiologisch abbaubare synthetische Polymere und Biopolymere, z.B. Alginat, Kollagen, Chitin, Chitosan, Polymilchsäure, Polyglycolsäure, Heparin, Hyaluronsäure, Polyrotaxan, Gelatine, Elastin, Fibrin, Laminin, Fibronectin etc., sowie Kombinationen davon. In einer speziellen Ausführungsform handelt es sich dabei um sterilisiertes azelluläres, d.h. von Zellen befreites, biologisches Material, z.B. Dünndarmsubmucosa (SIS) oder eine natürliche Kollagen- oder Chitin/Chitosan-Matrix.

Die Matrix oder das Trägersystem weist eine bestimmte Form auf, die für den beabsichtigten Verwendungszweck, z.B. als Implantat, besonders günstig ist. Die Form kann z.B. der Form des natürlichen Gewebes oder Organs entsprechen und/oder für die Erhaltung einer bestimmten dreidimensionalen Zellanordnung und/oder die Versorgung der auf oder in der Matrix befindlichen Zellen mit Nährstoffen und/oder für die Integration des Implantats bzw. der Zellen in den Körper vorteilhaft sein. In Abhängigkeit von Art und Funktion des jeweiligen geschädigten Gewebes bzw. Organs und der Art der verwendeten Zellen sind eine große Vielfalt geeigneter Formen im Stand der Technik bekannt. Die Matrix oder das Trägerystem kann fest, halbfest oder flüssig sein. Die erfindungsgemäß verwendeten Matrices oder Trägersysteme weisen die Form von Perlen, einschließlich Mikroperlen, eines Schaums, Gels, Hydrokolloids, einer Membran, eines Fadens, eines Netzes, eines Schwamms oder einer anderen porösen Struktur, eines textilen Gewebes, Vlieses oder eines natürlichen Organs oder Gewebes auf.

In einer Ausführungsform umfaßt das Gewebe- oder Organkultursystem ferner ein Bioadhäsiv. Dieses Bioadhäsiv kann dazu dienen, die Haftung der kultivierten Zellen auf der Matrix oder dem Träger zu verbessern und/oder die Verbindung eines implantierten Gewebe- oder Organsystems mit dem Gewebe eines Empfängers zu ermöglichen. In einer bevorzugten Ausführungsform basiert das Bioadhäsiv auf Fibrin und ist z.B. ein Fibrin-Kleber. Solche Fibrin-Kleber und äquivalente Biadhäsive sind auch im Handel erhältlich.

Die erfindungsgemäß hergestellten multizellulären Gewebe- und Organkultursysteme können zur Wiederherstellung oder Übernahme der Funktion geschädigter oder fehlender Gewebe oder Organe eines tierischen, vorzugsweise humanen, Individuums verwendet werden. Vorzugsweise stammen die pluripoten oder multipotenten adulten Stammzellen, aus denen die zur Herstellung des multizellulären Gewebe- oder Organkultursystems verwendeten organoiden Körper hervorgehen, von dem Individuum mit einem geschädigten oder fehlenden Gewebe oder Organ selbst. Durch Verwendung eines solchen "autologen" Materials können die eingangs geschilderten Probleme mit Abstoßungsreaktionen und Krankheitsübertragungen von fremden Spendern vermieden werden.

Das geschädigte Gewebe oder Organ, dessen Funktion wiederhergestellt oder übernommen werden soll, kann grundsätzlich jedes Organ oder Gewebe sein, dessen spezifische Zelltypen mit dem vorliegenden Verfahren kultiviert werden können. Einige bevorzugte, jedoch keineswegs beschränkende, Beispiele umfassen Haut, Nervengewebe, Muskelgewebe, Bindegewebe, Knorpelgewebe, neuromuskuläres Gewebe, Knochengewebe, Drüsengewebe, gastrointestinales Gewebe, Gefässe, Herz, Leber, Niere, Lunge Blutzellen etc.

Die häufigste Form der Verwendung der erfindungsgemäß hergestellten multizellulären Gewebe- und Organkultursysteme wird in der Verwendung als Implantat bestehen.

Ebenfalls möglich sind jedoch auch andere Verwendungen, insbesondere medizinische Verwendungen. In einer speziellen Ausführungsform wird das multizelluläre Gewebe- oder Organkultursystem extrakorporal in seiner Funktion als Gewebe- oder Organersatz verwendet. Ein konkretes Beispiel hierfür wäre die Verwendung eines künstlichen Nieren- oder Lebersystems zur Blutwäsche. Andere geeignete Verwendungen, z.B. zur Produktion gewebe- bzw. organspezifischer Substanzen werden für den Fachmann unschwer ersichtlich sein.

### FIGURENBESCHREIBUNG

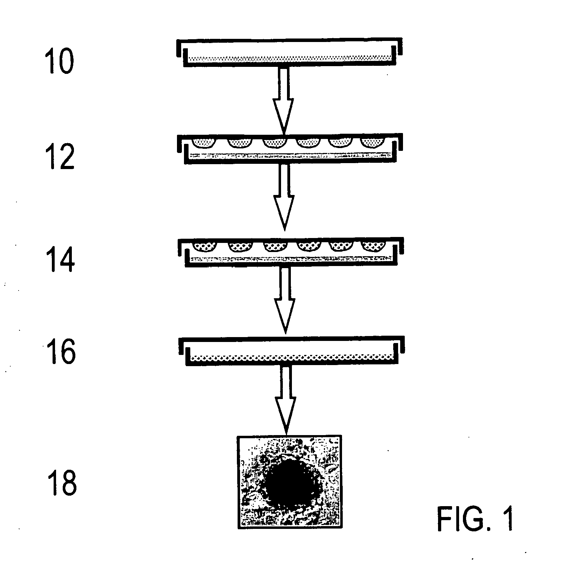

- Figur 1:: zeigt schematisch die Kultivierung der Stammzellen in Oberflächenkultur und in hängenden Tropfen sowie die Bildung und weitere Kultivierung von organoiden Körpern.

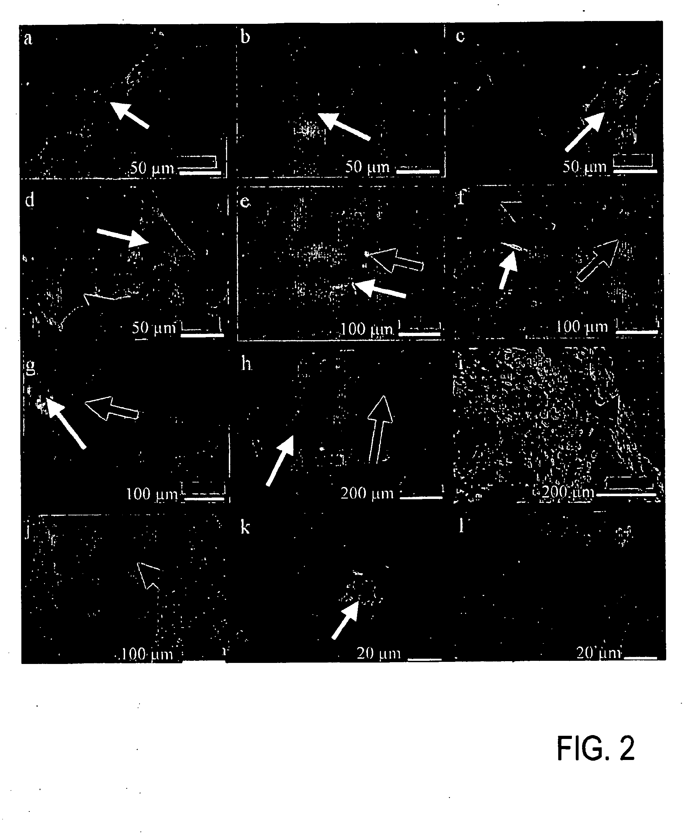

- Fig. 2: zeigt die Expression von Markern neuronaler Zellen, glialer Zellen und glatter Muskelzellen sowie von Amylase und Insulin in den differenzierten Zellen der organoiden Körper.
a,b: PGP 9.5-markierte Nervenzellen zeigen multipolare Ausläufer, welche zahlreiche Varikositäten zeigen. c,d: das Neurofilament-System (helle Pfeile, im Originalfoto grün markiert) reicht durch das Pericaryon in die cytoplasmatischen Ausläufer. In enger Nachbarschaft liegen GFAP-immunreaktive Gliazellen (dunkle Pfeile, im Originalfoto rot markiert). e,f: α-SMA-markierte Zellen (dunkle Pfeile, im Original rot) und NF-markierte Nervenzellen (helle Pfeile, im Original Grün) bilden ein primitives neuro-muskuläres Netzwerk (e), wobei Kontakte über beträchtlich lange Entfernungen etabliert werden (f). g: Immunfärbung von GFAP (dunkle Pfeile, im Original rot) und NF (helle Pfeile, im Original grün) in 3 Wochen alten OB mit Konzentrationen an Nerven- und Gliazellen. h: Immunfärbung von α-SMA (dunkle Pfeile, im Original rot) und NF (helle Pfeile, im Original grün) in 3 Wochen alten OBs in einem fortgeschrittenen Stadium der Bildung eines neuro-muskulären Netzwerks. i,j: in Querschnitten von 8 Wochen alten OBs wurden für NF immunreaktive Zellen in direkter Nachbarschaft von Zellen gefunden, die immunreaktiv für α-SMA waren, ähnlich wie in nativen Geweben. k: eine Untergruppe von Zellen zeigt eine positive Färbung für Amylase (helle Pfeile, im Original grün). 1: Eine weitere Zelluntergruppe enthält granuläre Vesikel mit Immunreaktivität für Insulin. Die Kerne sind mit DAPI kontrastgefärbt (im Original blau).

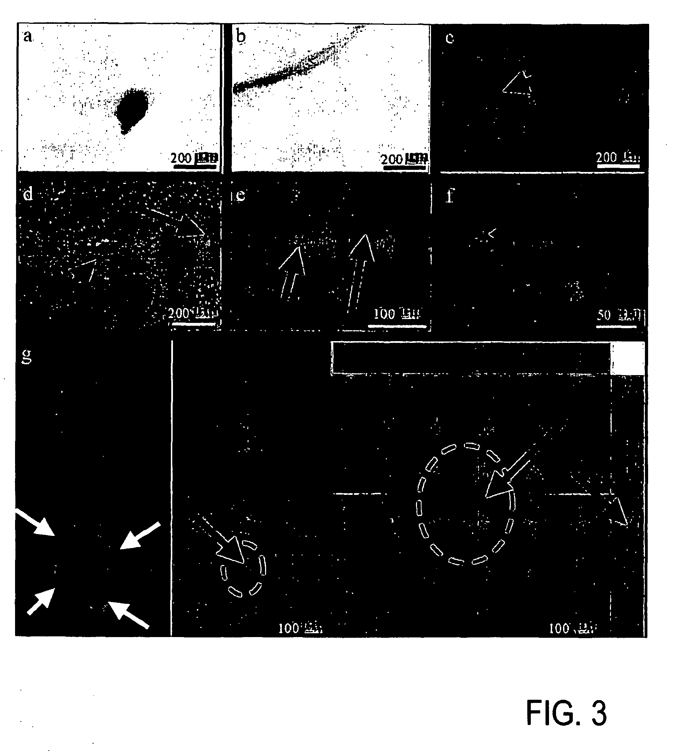

- Fig. 3: zeigt die Expression von extrazellulären Matrixkomponenten und Cytokeratinen.
a,b: Globuläre (a) und fibrilläre (b) Speicher von Proteoglycanen ergaben eine Färbung mit Alcianblau. c-e: Die globulären (c) und fibrillären (d) Bereiche sind immunreaktiv für das Knorpelmatrixprotein Kollagen II. Zwei individuelle Zellen (e) zeigen eine cytoplasmatische Markierung von Kollagen II. f: Zellen, die für Cytokeratine immunreaktiv sind, sind in Clustern angeordnet. g: konfokale Laserscanningmikroskopie eines OB. Die Kollagen II-Immunreaktivität (dunkle Pfeile, im Original rot markiert) nimmt zur Mitte des OB hin zu. Vigilinimmunreaktive Zellen (helle Pfeile, im Original grün markiert) sind hauptsächlich am äußeren Rand des OB lokalisiert, was deren hohe Translationsaktivität anzeigt. Die Kerne sind mit DAPI kontrastgefärbt.

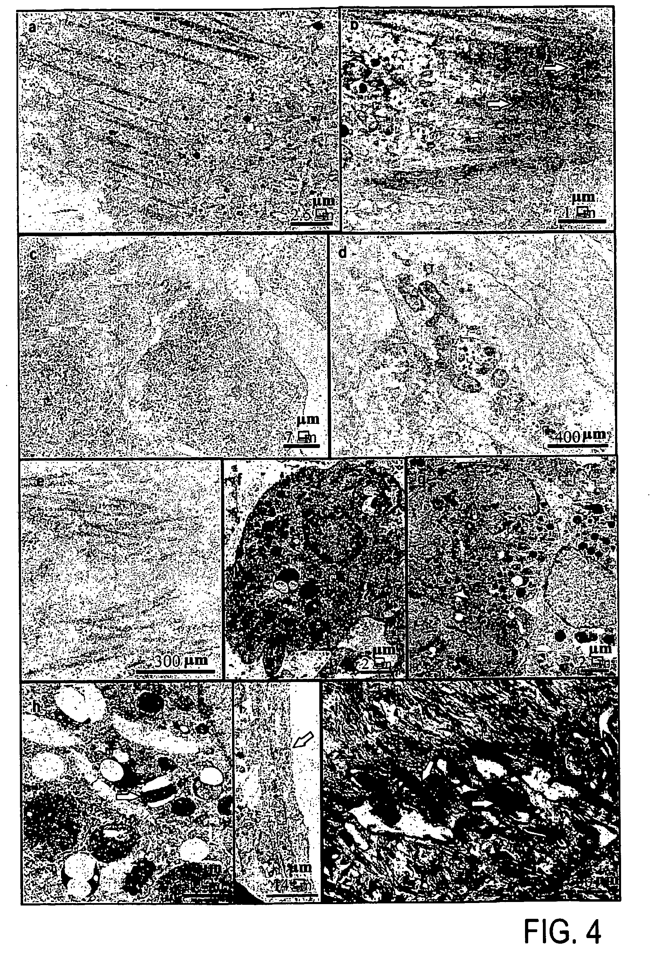

- Fig. 4: zeigt die Transmissionselektronenmikroskopie differenzierter OB.
α-c: glatte Muskelzellen mit Myofilamenten. Das Myofilamentsystem erstreckt sich über das Cytoplasma in disseminierten Bündeln (a) und zeigt typische dichte Körper (Pfeile) (b). Die Myoblasten zeigen sternförmige Zellausläufer, die ein verbindendes Netzwerk bilden (c). d: Zellausläufer mit einer Ansammlung von zahlreichen Vesikeln kleiner Größe, die höchstwahrscheinlich Nervenfaservarikositäten entsprechen. e: Kollagen- und Retikulumfasern. f-h: Sekretorische Zellen zeigen elektrodichte Vesikel. f). Häufig kontaktieren sekretorische Zellen einander, um acinusartige Strukturen zu bilden (g). Ein Untergruppe von sekretorischen Zellen enthält Vesikel (Pfeil) die Ultrastruktur-Merkmalen von endokrinen Granula entsprechen (h), z.B. Beta-Granula von insulinproduzierenden Zellen. 1: Beginn der Ausbildung einer Epitheloberfläche (Pfeil) in acht Wochen alten OBs. j: typische Zellkontakte zwischen Keratinozyten und Desmosomen (Pfeile)

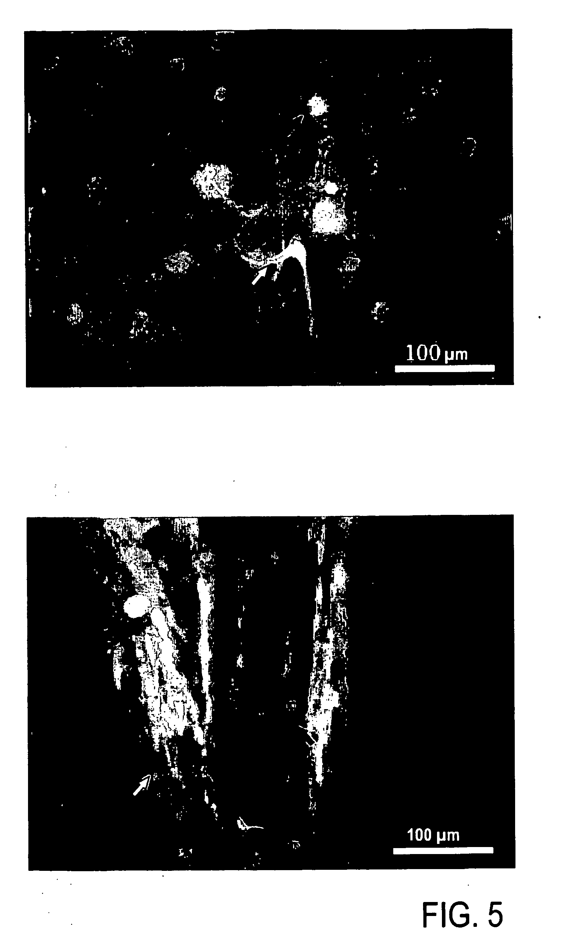

- Figur 5:: zeigt Nerven-Muskel-Komplexe aus der Gewebekultur; helle Pfeile (im Original grün) bedeuten Nervenzellen, dunkle Pfeile (im Original rot) bedeuten Muskelzellen. oben: Einzelzellen; unten Längsschnitte dreidimensionaler Zellkomplexe

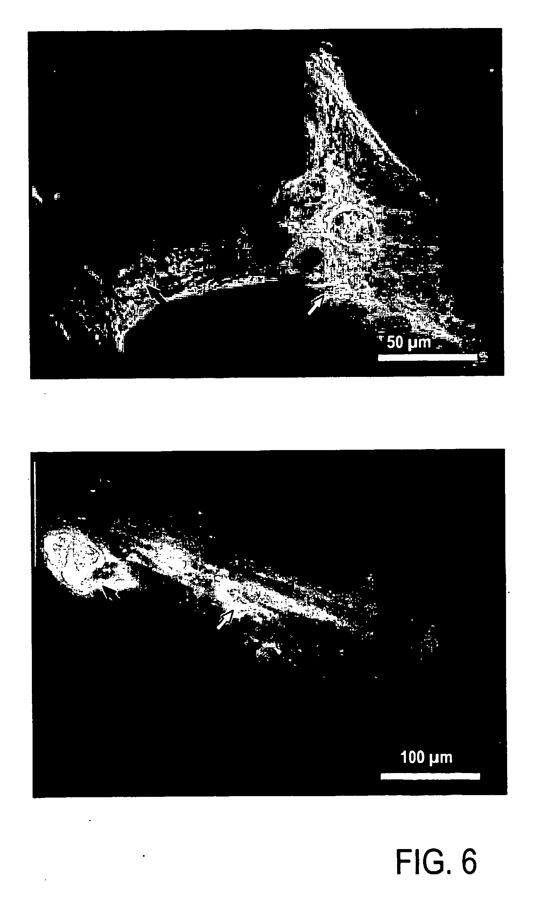

- Figur 6:: zeigt Nerven-Glia-Komplexe aus der Gewebekultur; helle Pfeile (im Original grün) bedeuten Nervenzellen, dunkle Pfeile (im Original rot) bedeuten Gliazellen. oben: Einzelzellen; unten Längsschnitte dreidimensionaler Zellkomplexe

Entsprechend dem in Figur 1 dargestellten Schema wird zur Gewinnung der Stammzellen exokrines Drüsengewebe, z.B. acinäres Gewebe - bevorzugt einer Speicheldrüse oder der Bauchspeicheldrüse (Pankreas) - mechanisch und enzymatisch zerkleinert in Kultur genommen (Schritt 10 in Figur 1). Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2 bis 3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Ähnliche Zellen sind unter gleichen Bedingungen aus dem Pankreas isoliert und beschrieben und als eine Art Myofibroblasten bzw. pankreatische Sternzellen bezeichnet worden (Bachem et al., 1998). Im Gegensatz zu den Zellen der vorliegenden Erfindung konnte eine uneingeschränkte Teilungsfähigkeit jedoch nicht beobachtet werden. Weiterhin konnten diese Zellen auch nicht unbegrenzt passagiert werden, ohne an Vitalität zu verlieren.

In einem zweiten Schritt (12) werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, so dass die Tropfen nach unten hängen.

Durch diese Art der Kultivierung bilden sich innerhalb von 48h die als organoide Körper bezeichneten Zellaggregate (14), die für ungefähr 6 Tage in eine Suspensionskultur umgesetzt werden (16). Die Teilansicht (18) aus Figur 1 zeigt eine mikroskopische Aufnahme eines solchen organoiden Körpers.

Die in Suspensionskultur wachsenden Organoid Bodies bilden neue Organoid Bodies, die auch bei Einzelzellen die Bildung von neuen Organoid Bodies induzieren. Die Zellen sind sowohl als Organoid Bodies als auch als Einzelzellen einfrierbar und behalten dabei ihre Vitalität und ihr Differenzierungspotenzial.

Die Figuren 2-4 zeigen mikroskopische und elektronenmikroskopische Aufnahmen von differenzierten Zellen, die aus solchen Organoid Bodies oder organoiden Körpern erhalten wurden.

### Dabei konnte z.B. die Bildung eines neuro-muskulären Netzwerks beobachtet werden:

Aus OBs erhaltene Zellen exprimierten stark α-SMA ("smoothmuscle-actin") (Fig. 2e-f). Die Anwesenheit verbreiteter Bündel von Myofilamenten, welche sich über das Cytoplasma erstreckten, wurde durch Elektronenmikroskopie bestätigt (Fig. 4a-c). Ferner wurden Zellen identifiziert, welche für den pan-Neuronenmarker PGP 9.5 und für Neurofilamente (NF) immunreaktiv waren. Das Neurofilamentsystem reichte vom Pericaryon in die radialen cytoplasmatischen Ausläufer (Fig. 2c,d). PGP 9.5-immunreaktive Zellen zeigten zahlreiche Varikositäten entlang ihrer verzweigten Ausläufer (Fig. 2a,b, 4d) und entsprachen damit typischen morphologischen Merkmalen autonomer Nervenfasern. Zellen, die für GFAP ("glial fibrillary acidic protein") immunreaktiv waren, befanden sich in enger Nachbarschaft zu Zellen, die neuronale Marker exprimierten (Fig. 2c,d). Häufig erstreckten sich die filamentösen Proteine nicht durch das gesamte Cytoplasma, sondern waren auf Gebiete neben den Nervenzellen beschränkt. Ferner waren glatte Muskelzellen und Nervenzellen nicht willkürlich verstreut, sonden bildeten zusammenhängende Netzwerke mit unschwer unterscheidbaren Verbindungen (Fig. 2e,f). Nervenfaserausläufer erstreckten sich über beträchtlich große Entfernungen, um benachbarte glatte Muskelzellen als ihre mutmaßlichen Ziele zu kontaktieren. Somit zeigten die beiden Zelltypen aufgrund ihrer topographischen Anordnung Merkmale eines primitiven neuromuskulären Netzwerks. Bei 3 Wochen alten OBs wurde eine beginnende Bildung von gewebeähnlichen Strukturen festgestellt (Fig. 2g-j). Hier wurde ein Cluster von faserförmigen Nervenzellen in Kontakt mit Gliazellen gefunden (Fig. 2g) oder weiter entwickelt zu einem dreidimensionalen neuro-muskulären Netzwerk (Fig. 2h), was in Querschnitten von 8 Wochen alten OBs bestätigt wurde (Fig. 2i,j).

### Nachweis der Expression exokriner und endokriner pankreatischer Proteine:

Immunhistochemische Anfärbungen zeigten, dass zelluläre Untergruppen positiv für Amylase waren (Fig. 2k). Das Immunreaktionssignal war auf klar unterscheidbare Vesikel inerhalb des apikalen Cytoplasmas beschränkt. Darüber hinaus waren die meisten Zellcluster, die für Amylase immunreaktiv waren, in Kreisen angeordnet, wobei die sekretorischen Vesikel eine Position zur Mitte hin aufwiesen, welches eine morphologische Anordnung ähnlich der von exokrinen Pankreas-Acini ist. Andere zelluläre Untergruppen zeigten Immunreaktivität für Insulin (Fig. 2l). Ähnlich wie bei den Amylase-positiven Zellclustern, war das sekretorische Produkt in vesikulären Strukturen gespeichert, die an einem Zellpol konzentriert waren. Die Anwesenheit sekretorischer Zellen wurde durch Elektronenmikroskopie bestätigt, welche dicht verteilte elektrodichte Partikel zeigte, wie sie charakteristisch für exkretorische oder inkretorische Funktionen sind (Fig. 4f-h).

### Ebenfalls beobachtet wurde eine Differenzierung in chondrogene Zellen und Epithelzellen:

Nach einer Wachstumsperiode von zwei Monaten zeigten OBs chondrogene Eigenschaften. Eine Alcianblaufärbung offenbarte Bereiche mit hohen Konzentrationen an Proteoglycanen (Chondroitinsulfat), die entweder als globuläre (Fig. 3a) oder fibrilläre (Fig. 3b) Ablagerungen vorkamen. Immunhistochemische Anfärbungen mit Antikörpern, die gegen das Knorpelmatrixprotein Kollagen II gerichtet waren, belegten zusätzlich die chondrogene Aktivität innerhalb dieser globulären (Fig. 3c) und fibrillären (Fig. 3d) Bereiche. Die Immunreaktivität war am höchsten in der Mitte der zellulären Aggregate, die am wahrscheinlichsten Bereichen von sich entwickelnder extrazellulärer Knorpelmatrix entsprach. Diese Beobachtung wurde durch konfokale Mikroskopie bestätigt (Fig. 3g): während die Menge der Kollagen-Speicher zur Mitte der zellulären Aggregate hin zunahm, waren die Randbereiche durch aktiv translatierende Zellen charakterisiert, wie durch deren hohe Vigilin-Expression demonstriert, die gewöhnlich bei Zellen mit aktiver Translationsmaschinerie, z.B. Kollagensynthetisierenden Chondrozyten oder in Fibroblasten während der Chondroinduktion, gefunden wird. Typische einzelne Kollagen II-translatierende Chondrozyten wurden auch in auswachsenden Zellen von OBs beobachtet, die eine Kollagen IIhaltige Matrix produzierten, welche die Einzelzellen umgab (Fig. 3e). Eine Ultrastruktur-Untersuchung dieser Bereiche konnte klar ein Netzwerk von Retikulumfasern und Kollagenfasern zeigen, wobei die letzteren durch ihre charakteristisches Bandenmuster identifiziert wurden (Fig. 4e). Einige Zellen exprimierten neben mesenchymalen Markern auch mehrere Cytokeratine, was deren Potenzial zur Differenzierung in Epithelzellen anzeigt. Jedoch wurden Zellen, die für Cytokeratine immunreaktiv waren, weniger häufig gefunden als Zellen, die Marker von glatten Muskelzellen und Neuronen exprimierten. Typischerweise waren sie in Clustern angeordnet, die innerhalb der OBs disseminiert waren (Fig. 3f). Durch elektronenmikroskopische Untersuchungen wurden typische Zellkontakte zwischen Keratinozyten gefunden (Fig. 4j) und bei 8 Wochen alten OBs wurden Epithelzellen auf der Oberfläche gefunden, welche aus dem Zellkulturmedium in die Luft wuchs.

Insgesamt konnten bisher z.B. folgende Marker für spezifische Zellen positiv getestet werden: PGP 9.5 und NF für Nervenzellen, S 100 und GFAP für Gliazellen, SMA für Muskelzellen (bzw. Myofibroblasten), Kollagen Typ II für Knorpelzellen, Amylase und Trypsin für exokrine Drüsenzellen, Insulin für endokrine Drüsenzellen, Vigilin für stark translatierende Zellen und Cytokeratin für epidermale Zellen. Neben den lichtmikroskopischen Untersuchungen konnten auch elektronenmikroskopisch verschiedene Zelltypen morphologisch charakterisiert werden, sowie Zell-Zell-Kontakte als Zeichen für zelluläre Interaktionen gefunden werden.

Es wurden bisher u.a. glatte Muskelzellen, Neuronen, Gliazellen, Epithelzellen, Fettzellen, Herzzellen, Nierenzellen, Fibroblasten (z.B. Haut- und Sehnenfibroblasten), Chondrozyten, endokrine und exokrine Drüsenzellen und damit Zelltypen aller drei Keimblätter morphologisch/histologisch und/oder immunochemisch in diesen organoiden Körpern nachgewiesen.

In den folgenden, nicht-beschränkenden Beispielen soll die vorliegende Erfindung näher erläutert werden.

Die allgemeinen Arbeitsanweisungen, wie sie für Verfahren zur Kultivierung von Säugerzellen, insbesondere humanen Zellen, gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| | |
|---|---|
| HEPES-Stammlösung (pH 7.6) | 2.3 83 g HEPES auf 100 ml A. *bidest.* |
| HEPES-Eagle-Medium (pH 7,4) | 90 ml Modified Eagle Medium (MEM) |
| | 10 ml HEPES-Stammlösung |
| Isolationsmedium (pH 7,4) | 32 ml HEPES-Eagle-Medium |
| | 8 ml 5% BSA in A. bidest. |
| | 300 µl 0,1 M CaC12 |
| | 100 µl Trasylol (200.000 KIE) |
| Digestionsmedium (pH 7,4) | 20 ml Isolationsmedium |
| | 4 ml Kollagenase (Kollagenase NB 8 von Serva) |
| Inkubationsmedium | Dulbecco's Modified Eagle Medium (DMEM) |
| Nährmedium | Dulbecco's Modified Eagle Medium (DMEM) |
| | DMEM + 4500 mg/l Glucose |
| | + L-Glutamin |
| | - Pyruvat |
| | + 20 % FKS (inaktiviert) + 1 ml/100 ml Pen/Strep (100000 E/10000 µg/ml) |
| | oder |
| | DMEM + 10 % Eigenplasma + 1 ml/100 ml Pen/Strep, |
| | vor Gebrauch auf 37°C erwärmen |
| Differenzierungsmedium | 380 ml DMEM |
| | 95 ml 30 min bei 54 °C inaktiviertes FKS |
| | 5 ml Glutamin (GIBCO BRL) |
| | 5 ml (3,5 µl ß-Mercaptoethanol auf 5 ml PBS) |
| | 5 ml Non-essential amino acids (GIBCO BRL) |
| | 5 ml Penicillin/Streptomycin (GIBCO BRL) |
| | (10000 E/10000 µg/ml) |

Statt fötalem Kalbserum (FKS) im Nährmedium und Differenzierungsmedium kann gegebenenfalls auch Serum oder Plasma einer anderen geeigneten Spezies, z.B. Humanplasma oder, weniger bevorzugt, Humanserum, verwendet werden. Vorzugsweise wird Eigenplasma oder, weniger bevorzugt, Eigenserum des tierischen oder menschlichen Gewebedonors verwendet. Dies ist insbesondere dann von Bedeutung, wenn der Gewebedonor mit dem späteren Empfänger der Stammzellen oder davon abgeleiteten differenzierten Zellen identisch ist. Eine solche autologe Behandlung ist zur Verhinderung einer eventuellen Abstoßungsreaktion bevorzugt.

Das Nährmedium kann als Basismedium statt des verwendeten DMEM-Mediums auch ein anderes für die Kultivierung von eukaryotischen Zellen, insbesondere Säugerzellen, bekanntes geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Die folgenden Beispiele 1 bis 3 beschreiben Arbeitsprotokolle zur Isolierung und Kultivierung adulter pluripotenter Stammzellen aus acinärem Gewebe des Pankreas bzw. aus acinärem und tubulösem Gewebe der Speicheldrüse.

### BEISPIEL 1

Zur Isolierung und Kultivierung von humanen adulten Stammzellen wurde humanes Gewebe von erwachsenen Patienten unmittelbar nach einem chirurgischen Eingriff erhalten und sofort aufgearbeitet. Aus dem chirurgisch entfernten Gewebe, z.B. Pankreasgewebe, wurde gesundes Gewebe abgetrennt und in Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland), aufgenommen (bei 20 °C, geringerer Stoffwechsel). Das Pankreasgewebe wurde mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37°C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml- und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 50-100 g) und weiter gereinigt durch Waschen in Inkubationsmedium, enthaltend 24,5 mM HEPES (pH 7,5), 96 mM NaCl, 6 mM KCl, 1 mM MgCl₂, 2, 5 mM NaH₂PO₄, 0, mM CaCl₂, 11,5 mM Glucose, 5 mM Natriumpyruvat, 5 mM Natriumglutamat, 5 mM Natriumfumarat, 1 % (Vol./Vol.) modifiziertes Eagle-Medium, 1 % (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5 % CO₂ kultiviert. Das acinäre Gewebe starb dabei schnell (innerhalb von zwei Tagen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium.

### BEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde transduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland) injiziert. Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel) und das Pankreasgewebe mit einer Schere sehr fein zerkleinert und verarbeitet wie in Beispiel 1 beschrieben.

### BEISPIEL 3

Die Isolierung und Kultivierung aus exokrinem Gewebe der Ohrspeicheldrüse erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

Falls die Stammzellen, die wie oben beschrieben aus exokrinem Drüsengewebe hergestellt wurden, direkt oder indirekt (Erzeugung von organoiden Körpern bzw. differenzierten Zellen daraus) für humantherapeutische Zwecke genutzt werden sollen, sind aus Sicherheitsgründen noch verschiedene Bedingungen zu erfüllen, um eine Gefährdung des zu behandelnden Patienten auszuschließen, insbesondere:
- Verwendung von humanem Serum, vorzugsweise Eigenplasma des Patienten, an Stelle von FKS, erforderlichenfalls Aufreinigung des Serums bzw. Plasmas
- Ausschluss jeder tierischen Quelle von weiteren Medienzusätzen
- höchste Reinheit aller Substanzen, Sterilität von Geräten und Umgebung
- Sterilität und Reinheit der Stammzellkultur durch mehrmaliges Passagieren der Stammzellen und Kontrolle auf Kontamination durch Mycoplasmen oder andere Mikrorganismen
- sorgfältige Überprüfung des Ausgangsgewebes und der Stammzellen auf Tumorgenizität.

Die folgenden Beispiele 4 und 5 beschreiben detailliert zwei Arbeitsprotokolle zur Herstellung von organoiden Körpern (Organoid Bodies) und differenzierten Zellen.

### BEISPIEL 4

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen, die hier Organoid Bodies (OB) genannt werden sollen, aus jeweils vier Deckeln in je eine bakteriologische Petrischale mit 5 ml Inkubationsmedium mit 20 % FKS überführt und für weitere 96 h kultiviert.

Die Organoid Bodies werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1 % Gelatine beschichtete 6 cm Petrischalen, in die 4 ml Differenzierungsmedium vorgelegt wurde und die anschließend mit je 6 Organoid Bodies beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 Organoid Bodies beschickt werden.' Daneben können auch 24-Mtilden-Mikrotiterplatten verwendet werden, die mit 0,1 % Gelatine beschichtet wurden und in die je 1,5 ml pro Mulde Differenzierungsmedium vorgelegt wurde und anschließend mit je 4 Organoid Bodies beschickt werden.

Derart kultiviert, ist die Differenzierungsfähigkeit der Zellen in den Organoid Bodies aktiviert und die Zellen differenzieren sich in Zellen der drei Keimblätter Mesoderm, Entoderm und Ektoderm. Die Zellen können sowohl als Organoid Bodies als auch als einzelne Zellen gelagert und kultiviert werden und behalten ihre Pluripotenz.

### BEISPIEL 5

Für die Induktion der Differenzierung wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen in Differenzierungsmedium mit der oben angegebenen Zusammensetzung überführt und auf eine Dichte von etwa 3 x 10⁴ Zellen/ml eingestellt; z.B. durch Trypsinbehandlung einer Stammzellkultur in Nährmedium, 5-minütige Zentrifugation bei 1000 UpM und Resuspendierung des erhalteerhaltenen Pellets in Differenzierungsmedium und Verdünnung soweit erforderlich.

Anschließend wurden mit einer 20-µl-Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die Organoid Bodies (OB), aus jeweils vier Deckeln in je eine bakteriologische Petrischale mit 5 ml Inkubationsmedium mit 20 % FKS überführt (Deckel schräg halten und die OBs mit etwa 2,5 ml Nährmedium abspülen) und für weitere 5-9 Tage, vorzugsweise 96 h, kultiviert.

Die Organoid Bodies wurden nun vorsichtig mit einer Pipette aufgesammelt und in mit 0,1 % Gelatine beschichtete Zellkulturgefäße mit Differenzierungsmedium überführt. Die OB vermehrten sich nun und wuchsen in zum Teil einzelnen Zelkolonien, die wieder vermehrt, vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium vorgelegt worden war, und diese mit je 6 Organoid Bodies beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 Organoid Bodies beschickt wurden, und Thermanox-Platten (Nalge Nonc International, USA) für elektronenmikroskopische Studien. Eine weitere Alternative waren 24-Mulden-Mikrotiterplatten, die mit 0,1 % Gelatine beschichtet wurden und in die je 1,5 ml pro Mulde Differenzierungsmedium vorgelegt wurde und die anschließend mit je 4 Organoid Bodies beschickt wurden.

In einer bevorzugten Ausgestaltung des Verfahrens wurden OBs etwa 7 Wochen lang in den gelatine-beschichteten 6-cm-Petrischalen kultiviert und danach wurden einzelne OBs mit dem Microdissector (Eppendorf, Hamburg, Deutschland) nach den Anweisungen des Herstellers ausgeschnitten und dann z.B. auf frische 6-cm-Petrischalen, Chamber Slides oder Thermanox-Platten überführt. In einer weiteren bevorzugten Ausgestaltung wurden einzelne OBs mit Pipettenspitzen durch leichtes Ansaugen abgelöst und transferiert, anschließend z.B. Beobachtung unter dem inversen Mikroskop.

### BEISPIEL 6

### Charakterisierung differenzierter Zellen in den organoiden Körpern

### 1. Immunohistochemie

Organoid Bodies, die mindestens 3 Wochen lang auf Chamber Slides kultiviert worden waren, sowie Querschnitte von "Langzeit"-OBs wurden zweimal in PBS gespült, fünf Minuten lang mit Methanol:Aceton (7:3), enthaltend 1 g/ml DAPI (Roche, Schweiz) bei -20°C fixiert und dreimal in PBS gewaschen. Nach Inkubation in 10%igem normalem Ziegenserum bei Raumtemperatur für 15 Minuten wurden die Proben über Nacht mit primären Antikörpern bei 4°C in einer Befeuchtungskammer inkubiert. Die primären Antikörper waren gegen das Proteingenprodukt 9.5 (PGP 9.5, polyklonaler Kaninchenantikörper, 1:400, Ultraclone, Insel Wight), Neurofilamente (NF-Pan-Cocktail, polyklonaler Kaninchenantikörper, 1:200, Biotrend, Deutschland), α-Smooth-muscle-actin (α-SMA, monoklonaler Mausantikörper, 1:100, DAKO, Dänemark), gliales fibrilläres saures Protein (GFAP, monoklonaler Mausantikörper, 1:100, DAKO, Dänemark), Kollagen II (monoklonaler Mausantikörper II-II-6B3, 1:20, Developmental Studies Hybridoma Bank, University of Iowa, USA), Vigilin FP3 (1:200, Kügler et al., 1996), Cytokeratine (Pan Cytokeratin, monoklonaler Mausantikörper, 1:100, Sigma, USA), Alpha-Amylase (polyklonaler Kaninchenantikörper, 1:100, Calbiochem, Deutschland) und Insulin (monoklonaler Mausantikörper, 0,5 g/ml, Dianova, Deutschland) gerichtet. Nach dreimaligem Spülen mit PBS wurden Objektträger 45 Minuten lang bei 37°C mit entweder Cy3-markiertem Antimaus-IgG oder FITCmarkiertem Antikaninchen-IgG (Dianova), beide 1:200 verdünnt, inkubiert. Die Objektträger wurden dreimal in PBS gewaschen, mit Vectashield Mounting Medium (Vector, USA) beschichtet und mit einem Fluoreszenzmikroskop (Axiosop Zeiss, Deutschland) oder mit einem konfokalen Laserscanningmikroskop (LSM 510 Zeiss, Deutschland) analysiert. Eine Alcianblaufärbung wurde mittels Standardverfahren durchgeführt.

### 2. Transmissionselektronenmikroskopie

OBs wurden auf Thermanox-Platten (Nalge Nonc International, USA) mindestens 3 Wochen lang kultiviert. An den Thermanox-Platten haftende Proben wurden durch Eintauchen in 0,1 M Cacodylat-Puffer, enthaltend 2,5 % Glutaraladehyd und 2 % Paraformaldehyd, bei pH 7,4 24 h lang inkubiert. Nach einer Nachfixierung in 1% OsO₄, "en bloc"-Anfärbung mit 2% Uranylacetat und Dehydrierung in reinen Alkoholen wurden die Proben in A-raldite eingebettet. Nach Entfernung der Thermanox-Platte wurden Semithin-Schnitte entweder tangential oder senkrecht zur eingebetteten Zellkultur vorgenommen und mit Methylenblau und Azur II geschnitten. Ultradünne Schnitte wurden aus den Regionen von Interesse ausgeschnitten, mit Bleicitrat angefärbt und unter einem Transmissionselektronenmikroskop (Phillips, EM 109) untersucht.

### BEISPIEL 7

Organoide Körper wurden wie oben beschrieben hergestellt und in Differenzierungsmedium ohne zusätzliche Differenzierungsfaktoren wachsen gelassen (etwa 2-4 Wochen) bis neuromuskuläre Netzwerke beobachtet werden konnten. Fig. 5 zeigt solche Nerven-Muskel-Komplexe aus der Gewebekultur als Einzelzellen und als Längsschnitte dieser dreidimensionalen Zellkomplexe. Diese Nerven-Muskel-Komplexe können in den organoiden Körpern weiterentwickelt werden oder aus den organoiden Körpern entnommen und separat weiterkultiviert und entwickelt werden. Die weitere Kultivierung kann in einem herkömmlichen Kulturgefäß oder bereits in einem Trägersystem oder einer Matrix wie oben beschrieben, vorzugsweise auf Kollagen-Basis, durchgeführt werden. Die letztere Verfahrensführung erleichtert eine spätere Implantation der Gewebe- oder Organkultursysteme.

### BEISPIEL 8

Organoide Körper wurden wie oben beschrieben hergestellt und in Differenzierungsmedium ohne zusätzliche Differenzierungsfaktoren wachsen gelassen (etwa 2-4 Wochen) bis Nerven-Glia-Komplexe beobachtet werden konnten. Fig. 6 zeigt solche Nerven-Glia-Komplexe aus der Gewebekultur als Einzelzellen und als Längsschnitte dieser dreidimensionalen Zellkomplexe. Diese Nerven-Glia-Komplexe können in den organoiden Körpern weiterentwickelt werden oder aus den organoiden Körpern entnommen und separat weiterkultiviert und entwickelt werden. Die weitere Kultivierung kann in einem herkömmlichen Kulturgefäß oder bereits in einem Trägersystem oder einer Matrix wie oben beschrieben, vorzugsweise auf Kollagen-Basis, durchgeführt werden. Die letztere Verfahrensführung erleichtert eine spätere Implantation der Gewebe- oder Organkultursysteme.

## Patentansprüche

1. Verfahren zur Herstellung dreidimensionaler multizellulärer Gewebe- und Organkultursysteme *in vitro* unter Verwendung multizellulärer Säugerzellaggregate, als organoide Körper bezeichnet, **dadurch gekennzeichnet, dass** die organoiden Körper durch Aggregation und Differenzierung pluripotenter adulter Stammzellen aus exokrinem Drüsengewebe gebildet werden und differenzierte Zellen aller drei Keimblätter enthalten und dadurch dass das Gewebe- oder Organkultursystem eine physiologisch verträgliche Matrix oder ein physiologisch verträgliches Trägersystem umfasst, wobei die Matrix oder das Trägersystem die Form von Perlen, einschließlich Mikroperlen, eines Schaums, Gels, Hydrokolloids, einer Membran, eines Fadens, eines Netzes, eines Schwamms oder einer anderen porösen Struktur, eines textilen Gewebes, Vlieses oder eines natürlichen Organs oder Gewebes aufweist.

2. Verfahren nach Anspruch 1, **dadurch gekennzeichnet, dass** es sich bei den Säugerzellaggregaten um humane Zellaggregate handelt.

3. Verfahren nach Anspruch 1 oder 2, **dadurch gekennzeichnet, dass** es sich bei dem exokrinen Drüsengewebe um acinäres Gewebe handelt.

4. Verfahren nach einem der Ansprüche 1-3, **dadurch gekennzeichnet, dass** die Zelltypen der organoiden Körper aus der Gruppe aus Osteoblasten, Osteoclasten, Chondrozyten, Adipozyten, Fibroblasten, Muskelzellen, Endothelzellen, Epithelzellen, hematopoetischen Zellen, sensorischen Zellen, endokrinen und exokrinen Drüsenzellen, Gliazellen, neuronalen Zellen, Oligodendrozyten, Blutzellen, Darmzellen, Herz-, Lungen-, Leber-, Nieren- oder Pankreaszellen ausgewählt sind.

5. Verfahren nach einem der vorhergehenden Ansprüche 1-4, **dadurch gekennzeichnet, dass** die Zelltypzusammensetzung der organoiden Körper durch Kultivierung in einem Medium, welches zelltyp-spezifische Differenzierungs- und/oder Wachstumsfaktoren enthält, festgelegt wurde.

6. Verfahren nach Anspruch 5, **dadurch gekennzeichnet, dass** die Wachstums- und/oder Differenzierungsfaktoren aus der Gruppe bestehend aus bFGF, VEGF, DMSO und Isoproterenol, Fibroblastenwachstumsfaktor 4 (FGF4), Hepatozyten-Wachstumsfaktor (HGF), TGF-betal, EGF, KGF, Retinsäure, beta-NGF, BMP-4 und Activin-A ausgewählt sind.

7. Verfahren nach einem der Ansprüche 1-6, **dadurch gekennzeichnet, dass** zwei oder mehr der verschiedenen Zelltypen der organoiden Körper gewebe- oder organähnliche Strukturen bilden.

8. Verfahren nach Anspruch 7, **dadurch gekennzeichnet, dass** zwei oder mehr der verschiedenen Zelltypen der organoiden Körper neuromuskuläre Strukturen oder Glia-Nervenzell-Strukturen oder Hautzellstrukturen bilden.

9. Verfahren nach einem der Ansprüche 1-8, **dadurch gekennzeichnet, dass** die gewebe -oder organähnlichen Strukturen aus den organoiden Körpern entnommen und dann unter geeigneten Bedingungen zur Aufrechterhaltung und/oder Entwicklung der Strukturen weiter kultiviert werden bis eine gewünschte Zellmenge und/oder ein gewünschtes Entwicklungsstadium erreicht ist.

10. Verfahren nach Anspruch 9, **dadurch gekennzeichnet, dass** die Entnahme der gewebe -oder organähnlichen Strukturen aus den organoiden Körpern mit Hilfe langsamer Mikromanipulationstechnik erfolgt.

11. Verfahren nach Anspruch 9 oder 10, **dadurch gekennzeichnet, dass** die weitere Kultivierung in Gegenwart von Wachstums- und/oder Differenzierungsfaktoren, oder in Cokultur mit differenzierten Zellen oder Gewebestücken des gewünschten Zielgewebes oder Nachbargewebes geschieht.

12. Verfahren nach einem der vorhergehenden Ansprüche, **dadurch gekennzeichnet, dass** zur Herstellung der multizellulären Gewebe- oder Organkultursysteme vollständige organoide Körper eines Entwicklungsstadiums oder Teile davon mit vollständigen organoiden Körpern eines anderen Entwicklungsstadiums oder Teilen davon und/oder davon abgeleiteten differenzierten Einzelzellen und/oder undifferenzierten adulten Stammzellen gemeinsam kultiviert werden.

13. Verfahren nach Anspruch 12, **dadurch gekennzeichnet, dass** die genannten Teile der organoiden Körper gewebe- oder organartige Strukturen einschließen.

14. Verfahren nach einem der vorhergehenden Ansprüche, **dadurch gekennzeichnet, dass** die Matrix oder das Trägersystem im Körper abbaubar ist.

15. Verfahren nach Anspruch 14, **dadurch gekennzeichnet, dass** die Matrix oder das Trägersystem azelluläre Materialien umfassen, die aus der Gruppe aus Dünndarmsubmucosa (SIS), Alginat, Kollagen, Chitin, Chitosan, Polymilchsäure, Polyglycolsäure, Heparin, Hyaluronsäure, Polyrotaxan, Gelatine, Elastin, Fibrin, Laminin, Fibronectin ausgewählt sind.

16. Verfahren nach einem der Ansprüche 1-15, **dadurch gekennzeichnet, dass** das Gewebe- oder Organkultursystem ferner ein Bioadhäsiv umfaßt.

17. Verfahren nach Anspruch 16, **dadurch gekennzeichnet, dass** das Bioadhäsiv auf Fibrin basiert, z.B. ein Fibrin-Kleber ist.

## Claims

1. A method for the *in vitro* production of three-dimensional multicellular tissue and organ culture systems using multicellular mammalian cell aggregates, referred to as organoid bodies, **characterized in that** the organoid bodies were formed by aggregation and differentiation of pluripotent adult stem cells from exocrine glandular tissue and contain differentiated cells of all three germ layers and **in that** the tissue or organ culture system comprises a physiologically compatible matrix or a physiologically compatible carrier system, wherein the matrix or the carrier system has the form of beads, including microbeads, of a foam, gel, hydrocolloid, a membrane, a thread, a net, a sponge or another porous structure, a textile fabric, fleece or of a natural organ or tissue.

2. The method according to claim 1, **characterized in that** the mammalian cell aggregates are human cell aggregates.

3. The method according to claim 1 or 2, **characterized in that** the exocrine glandular tissue is acinar tissue.

4. The method according to any one of claims 1-3, **characterized in that** the cell types of the organoid bodies are selected from the group of osteoblasts, osteoclasts, chondrocytes, adipocytes, fibroblasts, muscle cells, endothelial cells, epithelial cells, hematopoietic cells, sensory cells, endocrine and exocrine glandular cells, glia cells, neuronal cells, oligodendrocytes, blood cells, intestinal cells, cardiac cells, lung cells, liver cells, kidney cells or pancreatic cells.

5. The method according to any one of the preceding claims 1-4, **characterized in that** the cell type composition of the organoid bodies was determined by cultivation in a medium containing cell-type-specific differentiation and/or growth factors.

6. The method according to claim 5, **characterized in that** the growth and/or differentiation factors are selected from the group consisting of bFGF, VEGF, DMSO and isoproterenol, fibroblast growth factor 4 (FGF4), hepatocyte growth factor (HGF), TGF beta 1, EGF, KGF, retinoic acid, beta NFG, BMP 4 and activin A.

7. The method according to any one of claims 1-6, **characterized in that** two or more of the different cell types of the organoid bodies form structures, which are similar to tissues or organs.

8. The method according to claim 7, **characterized in that** two or more of the different cell types of the organoid bodies form neuromuscular structures or glia-nerve cell structures or skin cell structures.

9. The method according to any one of claims 1-8, **characterized in that** the structures that are similar to tissues or organs are removed from the organoid bodies and then cultivated further under suitable conditions for maintaining and/or developing the structures until a desired cell amount and/or a desired development stage has been attained.

10. The method according to claim 9, **characterized in that** the removal of the structures that are similar to tissues or organs from the organoid bodies takes place with the aid of a slow micromanipulation technique.

11. The method according to claim 9 or 10, **characterized in that** the further cultivation takes place in the presence of growth and/or differentiation factors or in co-culture with differentiated cells or tissue pieces of the desired target tissue or adjacent tissue.

12. The method according to any one of the preceding claims, **characterized in that** in order to produce the multicellular tissue or organ culture systems complete organic bodies of a development stage or parts of them are cultivated together with complete organoid bodies of another development stage or parts of them and/or differentiated individual cells derived from them and/or undifferentiated adult stem cells.

13. The method according to claim 12, **characterized in that** the cited parts of the organoid bodies include structures that are similar to tissues or organs.

14. The method according to according to any one of the preceding claims, **characterized in that** the matrix or the carrier system is degradable in the body.

15. The method according to claim 14, **characterized in that** the matrix or the carrier system comprises acellular materials selected from the group of small intestinal submucosa (SIS), alginate, collagen, chitin, chitosan, polylactic acid, polyglycolic acid, heparin, hyaluronic acid, polyrotaxan, gelatin, elastin, fibrin, laminin, fibronectin.

16. The method according to any one of claims 1-15, **characterized in that** the the tissue or organ culture system furthermore comprises a bioadhesive.

17. The method according to claim 16, **characterized in that** the bioadhesive is based on fibrin, e.g., a fibrin adhesive.

## Revendications

1. Procédé de production de systèmes de culture de tissus et d'organes multicellulaires et tridimensionnels *in vitro* utilisant des agrégats de cellules de mammifères multicellulaires, appelés organoïdes, **caractérisé en ce que** les organoïdes sont formés par agrégation et différenciation de cellules souches adultes pluripotentes provenant du tissu des glandes exocrines et contiennent des cellules différenciées des trois feuillets embryonnaires et **en ce que** le système de culture des tissus ou d'organes comprend une matrice physiologiquement acceptable ou un système support physiologiquement acceptable, dans lequel la matrice ou le système support présente la forme de perles, y compris de microperles, d'une mousse, de gels, d'hydrocolloïdes, d'une membrane, d'un fil, d'un réseau, d'une éponge ou d'une autre structure poreuse, d'un tissu, voile textile ou d'un organe ou tissu naturel.

2. Procédé selon la revendication 1, **caractérisé en ce que** les agrégats de cellules de mammifères sont des agrégats de cellules humaines.

3. Procédé selon la revendication 1 ou 2, **caractérisé en ce que** le tissu de glandes exocrines est un tissu acinaire.

4. Procédé selon l'une des revendications 1 à 3, **caractérisé en ce que** les types de cellules des organoïdes sont choisis dans le groupe constitué par les ostéoblastes, ostéoclastes, chondrocytes, adipocytes, fibroblastes, cellules musculaires, cellules endothéliales, cellules épithéliales, cellules hématopoïétiques, cellules sensorielles, cellules de glandes endocrines et exocrines, cellules gliales, cellules neuronales, oligodendrocytes, cellules sanguines, cellules intestinales, cellules cardiaques, pulmonaires, hépatiques, rénales ou pancréatiques.

5. Procédé selon l'une des revendications précédentes 1 à 4, **caractérisé en ce que** la composition du type de cellules des organoïdes a été fixée par culture dans un milieu, qui contient des facteurs de différenciation et/ou de croissance spécifiques à un type de cellules.

6. Procédé selon la revendication 5, **caractérisé en ce que** les facteurs de différenciation et/ou de croissance sont choisis dans le groupe constitué par le bFGF, le VEGF, le DMSO et l'isoprotérénol, le facteur de croissance des fibroblastes 4 (FGF4), le facteur de croissance des hépatocytes (HGF), le TFG-bêta 1, l'EGF, le KGF, l'acide rétinoïque, le bêta-NGF, le BMP-4 et l'activine-A.

7. Procédé selon l'une des revendications 1 à 6, **caractérisé en ce que** deux des différents types de cellules des organoïdes ou plus forment des structures analogues à des tissus ou des organes.

8. Procédé selon la revendication 7, **caractérisé en ce que** deux des différents types de cellules des organoïdes ou plus forment des structures neuromusculaires ou des structures de cellules gliales nerveuses ou de cellules cutanées.

9. Procédé selon l'une des revendications 1 à 8, **caractérisé en ce que** les structures analogues à des tissus ou des organes sont prélevées sur les organoïdes et puis sont encore cultivées dans des conditions appropriées pour le maintien et/ou le développement des structures jusqu'à ce qu'une quantité de cellules voulue et/ou un stade de développement voulu soit atteint(e).

10. Procédé selon la revendication 9, **caractérisé en ce que** le prélèvement des structures analogues à des tissus ou des organes se fait sur les organoïdes à l'aide de la technique de micromanipulation lente.

11. Procédé selon la revendication 9 ou 10, **caractérisé en ce que** la poursuite de la culture se fait en présence de facteurs de croissance et/ou de différenciation, ou en co-culture avec des cellules différenciées ou des morceaux de tissus cibles ou de tissus voisins souhaités.

12. Procédé selon l'une des revendications précédentes, **caractérisé en ce que**, pour produire des systèmes de culture de tissus ou d'organes multicellulaires, on cultive conjointement des organoïdes complets d'un stade de développement ou des parties de ceux-ci avec des organoïdes complets d'un autre stade de développement ou des parties de ceux-ci et/ou de cellules isolées différenciées qui en sont dérivées et/ou de cellules souches adultes indifférenciées.

13. Procédé selon la revendication 12, **caractérisé en ce que** les parties citées des organoïdes incluent des structures de type tissus ou organes.

14. Procédé selon l'une des revendications précédentes, **caractérisé en ce que** la matrice ou le système support est dégradable dans le corps.

15. Procédé selon la revendication 14, **caractérisé en ce que** la matrice ou le système support comprend des matières acellulaires qui sont choisies dans le groupe constitué par la sous-muqueuse de l'intestin grêle (SIS), l'alginate, le collagène, la chitine, le chitosane, l'acide polylactique, l'acide polyglycolique, l'héparine, l'acide hyaluronique, le polyrotaxane, les gélatines, l'élastine, la fibrine, la laminine, la fibronectine.

16. Procédé selon l'une des revendications 1 à 15, **caractérisé en ce que** le système de culture de tissus ou d'organes comprend en outre un bioadhésif.

17. Procédé selon la revendication 16, **caractérisé en ce que** le bioadhésif est à base de fibrine, par exemple une colle à la fibrine.
